# EUROPEAN PATENT SPECIFICATION

(11) **EP 2 727 567 B1**
(45) Date of publication and mention of the grant of the patent: **06.01.2016**
(21) Application number: 12808215.3
(22) Date of filing: 29.06.2012
(51) Int. Cl.: A61F 13/15, A61F 13/49

(54) **METHOD FOR DISCHARGING ABSORPTIVE ARTICLE**
VERFAHREN ZUM ENTLADEN EINES SAUGFÄHIGEN ARTIKELS
PROCÉDÉ DE DÉCHARGEMENT D'ARTICLES ABSORBANTS

(30) Priority: 01.07.2011 JP 2011147780
(43) Date of publication of application: 07.05.2014
(73) Proprietor: Unicharm Corporation, Shikokuchuo-shi, Ehime 799-0111 (JP)
(72) Inventor: TAKAHASHI, Kazuhiko, Fukushima 963-6106 (JP); WATANABE, Tomohiro, Kanonji-shi Kagawa 769-1602 (JP); MIYAKI, Masanobu, Kanonji-shi Kagawa 769-1602 (JP)
(74) Representative: Peter, Julian
(86) International application number: PCT/JP2012/066747
(87) International publication number: WO 2013/005682

(56) References cited:
- DE-A1-102009 016 381
- JP-A- 2007 175 375
- JP-A- 2010 200 908
- US-A1- 2003 212 377
- US-A1- 2010 305 739

## Description

### [Technical Field]

The present invention relates to a method of ejecting an absorbent article used in a manufacturing line of the absorbent article.

### [Background Art]

Conventionally, an absorbent article such as a disposable diaper and a sanitary napkin is generally manufactured by using a manufacturing line in which an absorber, for example, is arranged sequentially on a continuous web conveyed by a belt conveyor, for example, and the manufacturing line includes a step of folding and a step of cutting the continuous web.

In such a manufacturing line, generally, an ejection mechanism is provided, by which an absorbent article for which a processing failure is determined is disengaged from the manufacturing line, and the absorbent article is ejected in the course of the manufacturing line. Because the manufacturing line operates at a high speed, normally, a plurality of preceding and succeeding absorbent articles including the absorbent article for which a processing failure is determined are ejected in succession.

Therefore, an ejection method by which the plurality of absorbent articles are ejected after printing a number and a symbol on the absorbent article is known (for example, see Patent Literature 1). Specifically, continuous numbers, or symbols such as a circle and a triangle are printed on the plurality of absorbent articles, and the plurality of absorbent articles on which the numbers or the symbols are printed are ejected.

According to such an ejection method, the operator can easily adjust the timing of ejection of the absorbent article such that among the plurality of absorbent articles that are ejected, the position of the absorbent article for which a processing failure is determined is generally at the center (for example, the third absorbent article when five absorbent articles are ejected).

### [Prior Art Document]

### [Patent Document]

[Patent Document 1] Japanese Patent Application Publication No. 2002-791187 (Page 3, Fig. 1)

### [Summary of the Invention]

However, the aforementioned conventional method of ejecting an absorbent article had the below-mentioned problem. That is, a certain number of absorbent articles for which a processing failure is not determined are also ejected together with the absorbent article for which a processing failure is determined, and because the ejected absorbent articles cannot be handled as products, an obstruction in improvement of the yield rate was a problem.

In particular, a continuous web is supplied without pausing a manufacturing line, while a web roll in use is joined to the following web roll, by using an accumulation device which accumulates and discharges a continuous web with expanding and contracting a distance between dancer rollers which add tension to a continuous web. However, although an absorbent article including a joint portion of continuous webs needs to be handled as a processing failure, it was a difficult problem to precisely eject only those absorbent articles having such a joint portion.

The present invention has been achieved in consideration of such circumstances, and an object thereof is to provide a method of ejecting an absorbent article that can precisely eject only absorbent articles having a joint portion and improve a yield rate.

The present invention is characterized by a method of ejecting absorbent articles (absorbent article PD) that is used in a manufacturing line of the absorbent articles, including: a step of arranging absorbers (absorber AB) on a conveyed continuous web (continuous web WB); a step of cutting the continuous web arranged with the absorbers at every predetermined interval in a conveyance direction (machine direction MD) of conveying the continuous web; and a step of providing a joint portion (joint portion P1) where a trailing end of the continuous web is joined to a starting end of a next continuous web, the method comprising: a step of controlling a timing of supplying the continuous web so as to locate the joint portion at a central portion (central portion CT) of the absorbent articles in a conveyance direction, based on a length of the continuous web from the joint portion up to a cutting location (cutting location P2) of the continuous web in the cutting step and a dimension (length L1) of the absorbent articles in a conveyance direction of the continuous web; and a step of disengaging only absorbent articles having at least the joint portion from the manufacturing line and ejecting the absorbent articles in the course of the manufacturing line.

### [Brief Description of Drawings]

[Fig. 1] Fig. 1 is a schematic diagram showing an entire configuration of a manufacturing line 10 of an absorbent article according to an embodiment of the present invention.
[Fig. 2] Fig. 2 is a partially expanded perspective view of an ejection device 130 according to the embodiment of the present invention.
[Fig. 3] Fig. 3 is a diagram illustrating an ejection operation flow of an absorbent article PD in the manufacturing line 10 according to the embodiment of the present invention.

### [Description of Embodiments]

Next, an embodiment of a method of ejecting an absorbent article according to the present invention is explained with reference to drawings. In the following description of the drawings, the same or similar reference numerals are used to designate the same or similar parts. It will be appreciated that the drawings are schematically shown and the ratio and the like of each dimension are different from the real ones.

Accordingly, specific dimensions should be determined in consideration of the explanation below. Moreover, among the drawings, the respective dimensional relations or ratios may differ.

### (1) Entire schematic configuration of manufacturing line

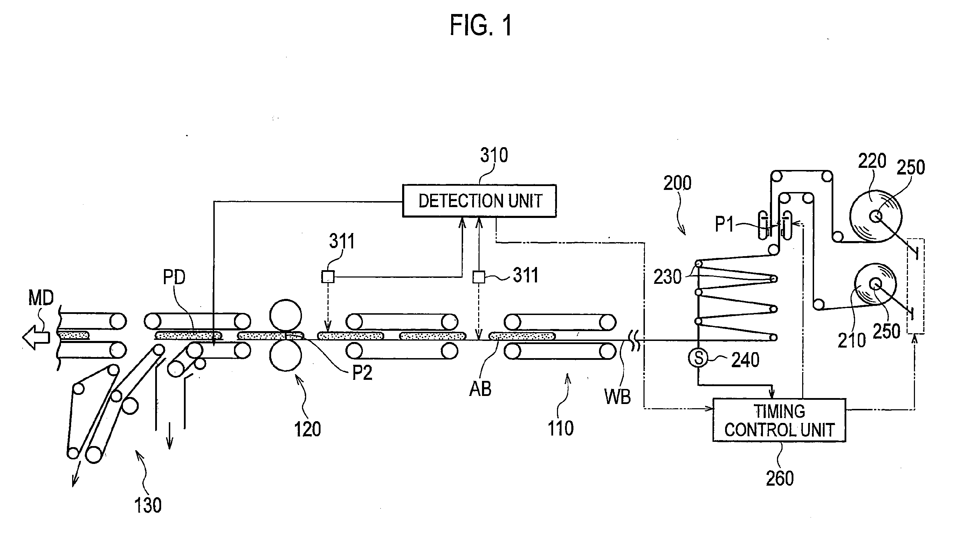

Fig. 1 is a schematic diagram showing an entire configuration of a manufacturing line 10 of an absorbent article according to the present embodiment. The manufacturing line 10 is configured by a plurality of devices configured to manufacture an absorbent article, specifically, an absorbent article PD having an absorber AB for absorbing liquids, such as a disposable diaper, a sanitary napkin, and a panty liner. The absorber AB is configured from pulp and an absorbent polymer, for example.

The manufacturing line 10 conveys a continuous web WB, which is a continuous body of a nonwoven fabric and a resin film, towards a machine direction MD, and arranges the absorber AB on the conveyed continuous web WB, and finally manufactures the absorbent article PD by folding and cutting the continuous web WB while conveying the continuous web WB.

That is, the manufacturing line 10 includes the steps of, for example, arranging the absorbers AB on the conveyed continuous web WB, and cutting the continuous web WB on which the absorbers AB are arranged, at every predetermined interval in the machine direction MD (conveyance direction) of conveying the continuous web WB.

Additionally, the continuous web WB is sequentially let out from a web roll 210 or a web roll 220. However, since one web roll in use needs to be switched to the following web roll, the manufacturing line 10 has the step of joining the continuous webs WB without pausing the manufacturing line 10. For example, the manufacturing line 10 has the step of forming a joint portion P1 where the trailing end of the continuous web WB, which is currently in use and let out from the web roll 210, is joined to the starting end of the continuous web WB, which is subsequently in use and let out from the web roll 220.

Specifically, the manufacturing line 10 has a conveyor 110, a product cutter 120, an ejection device 130, and an accumulation device 200. The manufacturing line 10 also has a detection control unit 310 and a camera 311 in order to detect the shapes and conditions of the conveyed continuous web WB and manufactured absorbent articles PD. Furthermore, the manufacturing line 10 has a timing control unit 260 configured to control the timing of supplying the continuous web WB.

The conveyor 110 is configured to convey the continuous web WB at a high speed along the machine direction MD, and is configured by a plurality of belt conveyors. Depending on the conveyance position, and the type of the continuous web WB to be conveyed and the absorbent article PD, a suction-type belt conveyor in which a plurality of through-holes are formed, and that has a suction function is used, if necessary.

The product cutter 120 cuts the continuous web WB on which the absorbent articles PD are arranged at every predetermined interval (that is, the product length of the absorbent article PD). The product cutter 120 is provided with a blade for cutting, and configured by a cutter roller rotating in the machine direction MD and an anvil roller facing the cutter roller and rotating similarly in the machine direction MD.

The ejection device 130 disengages absorbent articles PD having failure locations from the manufacturing line 10, and ejects the absorbent articles PD in the course of the manufacturing line 10. The absorbent articles PD having a joint portion P1 between the web roll 210 and the web roll 220 are also targeted for ejection.

The ejection device 130 is configured by a guide bar (not shown) which guides conveyed absorbent articles PD to an ejection route, and a suction-type belt conveyer, for example, that is arranged towards the ejection route. Particularly, in the present embodiment, in order to improve the yield rate of the absorbent article PD, the ejection device 130 is configured to eject either only the absorbent article PD having a failure location, or the absorbent article PD having a failure location, as well as the adjoining absorbent articles PD that exist before and after the absorbent article PD.

Moreover, as described above, since the absorbent articles PD having a joint portion P1 are also targeted for ejection, the ejection device 130 disengages only the absorbent articles PD having at least a joint portion P1 from the manufacturing line 10 and ejects the absorbent articles PD in the course of the manufacturing line 10.

The accumulation device 200 includes the web roll 210, the web roll 220, dancer rollers 230, and motors 250. The accumulation device 200 accumulates and discharges a continuous web WB by expanding and contracting a distance between a plurality of the dancer rollers 230.

More specifically, the plurality of the dancer rollers 230 are connected through a supporting plate 231, and add a predetermined tension to the conveyed continuous web WB.

The accumulation device 200 controls the accumulation amount of the continuous web WB that is supplied to the conveyor 110, by expanding and contracting a distance between the dancer rollers 230 in the horizontal direction (lateral direction in Fig. 1). In other words, as the distance is expanded, the accumulation amount of the continuous web WB increases, and as the distance between the dancer rollers 230 is contracted, the accumulation amount of the continuous web WB decreases. In order to detect the positions of the dancer rollers 230 which shift in such a manner, a sensor 240 is provided.

At the time of switching from the web roll 210 to the web roll 220 (or vice versa), the accumulation amount of the continuous web WB increases by expanding a distance between the dancer rollers 230. Accordingly, within the time of letting out the continuous web WB accumulated at the accumulation device 200, the web roll 210 can be switched to the web roll 220, in other words, the continuous webs WB can be joined together by forming a joint portion P1 without pausing the manufacturing line 10.

The accumulation device 200 also has the motors 250 and the timing control unit 260. The supplying speed of the continuous web WB is controlled based on the positions of the dancer rollers 230 detected by the sensor 240. The motors 250 and the timing control unit 260 decide the supplying speed of the continuous web WB based on the positions of the dancer rollers 230 detected by the sensor 240, and control the timing for forming the joint portion P1.

More specifically, the timing control unit 260 decides the supplying speed of the continuous web WB based on the positions of the dancer rollers 230, and decides the supplying speed of the continuous web WB while verifying whether or not the dancer rollers 230 have shifted to predetermined positions, thereby controlling the timing for forming the joint portion P1.

The manufacturing line 10, as mentioned above, has the detection control unit 310 and the camera 311 so as to detect the shapes and conditions of the conveyed continuous web WB and the manufactured absorbent articles PD. The detection control unit 310 is connected to the plurality of cameras 311 installed at a predetermined position on the manufacturing line 10 (for example, immediately after each step).

The detection control unit 310 directs the timing and ejection time for ejecting absorbent articles PD having failure locations to the ejection device 130, based on an image which shows the shapes and conditions of the continuous web WB or the absorbent articles PD recorded by the camera 311. Accordingly, it becomes possible to eject either only the absorbent articles PD having failure locations, or the absorbent articles PD having failure locations, as well as the adjoining absorbent articles PD that exist before and after the absorbent article PD. Additionally, the detection control unit 310 directs the timing for ejecting absorbent articles PD having a joint portion P1 to the ejection device 130.

### (2) Schematic configuration of ejection device of absorbent articles

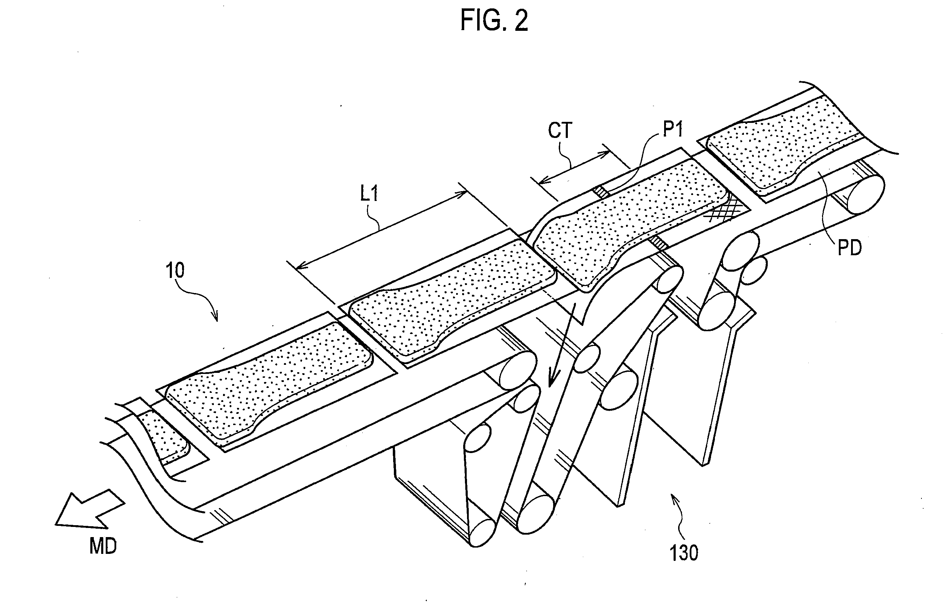

Fig. 2 is a partially enlarged perspective view of the ejection device 130. As shown in Fig. 2, the ejection device 130 disengages the absorbent articles PD having a joint portion P1 from the manufacturing line 10, and ejects the absorbent articles PD in the course of the manufacturing line 10. In the present embodiment, a double sided tape is used so as to form a joint portion P1, in other words, to join the trailing end of the continuous web WB which is currently in use to the starting end of the continuous web WB which is to be used subsequently. Moreover, although a heat seal, for example, may be used instead of the double sided tape so as to form a joint portion P1, it is preferable to quickly finish joining with the double sided tape or the like, as the required accumulation amount of the continuous web WB at the accumulation device 200 increases.

A joint portion P1 is formed at the central portion CT of an absorbent article PD. The central portion CT is a region within 30% or less of the dimension L1 of the absorbent article PD from the center of the absorbent article PD in the machine direction MD (conveyance direction). That is, the central portion CT is the region including 60% of the dimension L1 with the center of the absorbent article PD in the machine direction MD as the reference. Thus, the accumulation device 200 controls so as not to locate the joint portions P1 in a 20% region of the dimension L1 from the ends of absorbent articles PD in the machine direction MD.

The purpose of forming a joint portion P1 at the central portion CT is to prevent a joint portion P1 from straddling a plurality (two) of absorbent articles PD. When a joint portion P1 does not straddle a plurality (two) of absorbent articles PD, it is not necessarily required to form a joint portion P1 in a 60% region of the dimension L1 with the center of the absorbent article PD as a reference. For example, the central portion CT may be a region within about 80% of the dimension L1 with the center of the absorbent article PD in the machine direction MD as a reference.

As shown in Fig. 2, the absorbent article PD having a joint portion P1 is disengaged from the manufacturing line 10, and is guided to an ejection route (see an arrow in the figure). Absorbent articles PD with no joint portion P1 are conveyed to the following step.

### (3) Operation of ejecting absorbent articles

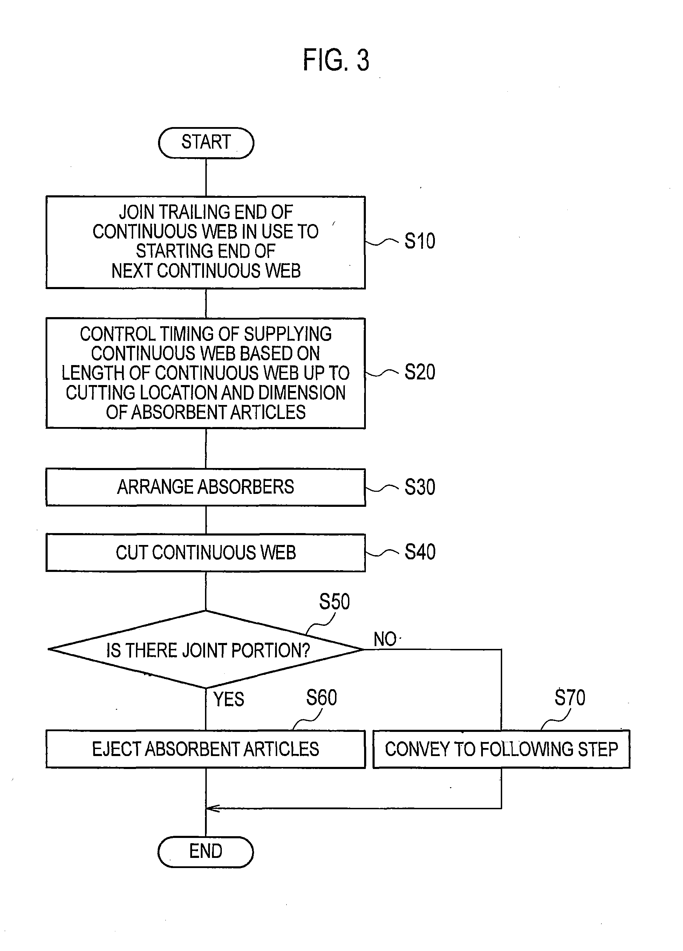

Fig. 3 illustrates the ejection operation flow of the absorbent article PD in the manufacturing line 10. Specifically, Fig. 3 illustrates the operation flow of the manufacturing line 10 relating to the ejection of absorbent articles PD having a joint portion P1.

As illustrated in Fig. 3, the accumulation device 200 forms a joint portion P1 by joining the trailing end of the continuous web WB which is currently in use (for example, continuous web WB let out from the web roll 210) to the starting end of the continuous web WB which is to be used subsequently (for example, continuous web WB let out from the web roll 220) (S10).

The accumulation device 200 controls the timing for supplying the continuous web WB to the conveyor 110 based on the length of the continuous web WB from a joint portion P1 up to a cutting location P2 of the continuous web WB (see Fig. 1) in the step of cutting the continuous web WB that is arranged with the absorbers AB, with the product cutter 120, and the dimension L1 of absorbent articles PD in the machine direction MD of the continuous web WB (S20).

Specifically, the accumulation device 200 controls the rotating speed of the motors 250 or the operation region of the dancer rollers 230 and thus controls the timing of supplying the continuous web WB so as to locate a joint portion P1 at the central portion CT of absorbent articles PD in the machine direction MD.

More specifically, the timing control unit 260 of the accumulation device 200 changes the accumulation amount of the continuous web WB by adjusting a distance between the dancer rollers 230 based on the length of the continuous web WB on the conveyance route from the location of a joint portion P1 up to the cutting location P2 shown in Fig. 1 and also the dimension L1 of absorbent articles PD. In other words, the accumulation device 200 calculates the accumulation amount of the continuous web WB so as to control the length of the continuous web WB from the location of a joint portion P1 up to the cutting location P2 shown in Fig. 1 and thus to locate the joint portion P1 at the central portion CT. Moreover, such a calculation can be carried out based on the length (for example, 10m) of the continuous web WB, the dimension L1 (for example, 0.5m), and the number and distance of the dancer rollers 230.

Subsequently, the manufacturing line 10 arranges the absorbers AB at every predetermined interval (dimension L1) therebetween on the continuous web WB conveyed by the conveyor 110 (S30). Additionally, the step of arranging the absorbers AB is subsequently carried out along with the steps S10 and S20 described above.

The manufacturing line 10 cuts the continuous web WB arranged with the absorbers AB by using the product cutter 120 at every predetermined interval in the machine direction MD of conveying the continuous web WB (S40).

Furthermore, the manufacturing line 10 determines whether or not the absorbent articles PD cut by the product cutter 120 have a joint portion P1 (S50). When the absorbent article PD has a joint portion P1, the manufacturing line 10 disengages the absorbent article PD from the manufacturing line 10 and ejects the absorbent article PD in the course of the manufacturing line 10 by using the ejection device 130 (S60). On the other hand, when absorbent articles PD have no joint portion P1, the manufacturing line 10 conveys the absorbent articles PD to the following step by using the conveyor 110 (S70).

As described above, the ejection device 130 can eject only the absorbent articles PD having a joint portion P1.

### (4) Operation and Effect

According to the manufacturing line 10 relating to the present embodiment, the timing for supplying the continuous web WB is controlled so as to locate a joint portion P1 at the central portion CT of the absorbent article PD in the machine direction MD (conveyance direction), based on the length of the continuous web WB up to the cutting location P2 and the dimension L1 of absorbent articles PD. Specifically, with the use of the accumulation device 200, the accumulation amount of the continuous web WB is controlled, and the supply of the continuous web WB is controlled so as to locate a joint portion P1 at the central portion CT.

Thus, a joint portion P1 is prevented from straddling and locating over a plurality (two) of absorbent articles PD, so that only the absorbent articles PD having joint portions P1 can be ejected. When a joint portion P1 is near the end in the machine direction MD (conveyance direction) of the absorbent articles PD, there may occur various failures (excessive wrinkles or bias, for example) caused due to the fact that the joint portion P1 straddles two absorbent articles PD, and although the joint portion P1 is excluded, the preceding (or succeeding) absorbent article PD of the absorbent article PD includes the joint portion P1, and as a result, it becomes inevitable to eject a plurality of absorbent articles PD, which is not preferable.

That is, according to the manufacturing line 10 relating to the present embodiment, the ejection of the plurality of absorbent articles PD having no failure locations can be avoided by precisely ejecting only the absorbent articles PD having a joint portion P1, and the yield rate of absorbent articles PD can improve.

In the present embodiment, the accumulation device 200, specifically, the plurality of dancer rollers 230 and the sensor 240 change the speed of supplying the continuous web WB, by controlling the timing of supplying the continuous web WB. Accordingly, while the accumulation device 200 is used in order to keep supplying the continuous web WB without pausing the manufacturing line 10, the supply of the continuous web WB can be controlled so as to locate a joint portion P1 at the central portion CT.

Additionally, even when the speed of manufacturing absorbent articles PD in the manufacturing line 10 changes at the time of switching from the web roll 210 to the web roll 220, or even when the roll diameters or weights of the web roll 210 and the web roll 220 are different from each other, the supply of the continuous web WB can be controlled so as to locate a joint portion P1 at the central portion CT.

In the present embodiment, the plurality of dancer rollers 230 are configured to expand and contract a distance between the dancer rollers 230 in the horizontal direction. Thus, even when a distance between the plurality of dancer rollers 230 is expanded and contracted, the plurality of dancer rollers 230 is unlikely to be affected by gravity and it is possible to locate a joint portion P1 at the central portion CT more precisely. Furthermore, this is particularly preferable when a load application is controlled by a servo motor, for example, so as to accurately add tension at the dancer rollers 230.

### (5) Other embodiments

So far, the present invention is disclosed through the above embodiments. However, it should not be interpreted that the statements and drawings constituting a part of the present disclosure limit the present invention. From this disclosure, a variety of alternate embodiments, examples, and applicable techniques will become apparent to one skilled in the art.

For a mechanism to locate a joint portion P1 at the central portion CT, for example, there is no need to use the dancer rollers 230, and the accumulation amount of the continuous web WB may be controlled by using a device other than the dancer rollers 230.

Additionally, the ejection device 130 having a guide bar and a suction-type belt conveyor is only one example, the ejection device for ejecting absorbent articles PD may be configured only by a guide bar or only by a suction-type belt conveyor.

Furthermore, in the manufacturing line 10 shown in Fig. 1, the steps of manufacturing absorbent articles PD are simplified. It is certainly possible that the manufacturing line 10 may include other various steps (for example, the step of arranging elastic members), depending on the kinds of the absorbent articles PD.

As described above, needless to say, the present invention includes various embodiments and the like not described here. Therefore, the technical range of the present invention is to be defined only by the inventive specific matter according to the adequate claims from the above description.

The contents of Japanese Patent Application No. 2011-147780 (filed on July 1, 2011) are all incorporated in the present specification by reference.

### [Industrial Applicability]

As described above, according to the present invention, it is possible to provide the method of ejecting an absorbent article that can improve a yield rate by precisely ejecting only the absorbent articles having a joint portion.

### [Reference Signs List]

10...manufacturing line
110...conveyor
120...product cutter
130... ejection device
200... accumulation device
210, 220...web roll
230...dancer roller
231...supporting plate
240...sensor
250...servo motor
260...control unit
310...detection control unit
311... camera
AB...absorber
P1...joint portion
P2...cutting location
PD...absorbent article
WB...continuous web

## Claims

1. A method of ejecting absorbent articles that is used in a manufacturing line of the absorbent articles, including: a step of arranging absorbers on a conveyed continuous web;
a step of cutting the continuous web arranged with the absorbers at every predetermined interval in a conveyance direction of conveying the continuous web; and
a step of providing a joint portion where a trailing end of the continuous web is joined to a starting end of a next continuous web, the method comprising:
a step of controlling a timing of supplying the continuous web so as to locate the joint portion at a central portion of the absorbent articles in a conveyance direction, based on a length of the continuous web from the joint portion up to a cutting location of the continuous web in the cutting step and a dimension of the absorbent articles in a conveyance direction of the continuous web; and
a step of disengaging only absorbent articles having at least the joint portion from the manufacturing line and ejecting the absorbent articles in the course of the manufacturing line.

2. The method of ejecting absorbent articles according to claim 1, wherein in the step of controlling the timing of supplying the continuous web, dancer rollers which add tension to the continuous web which is being conveyed, a sensor for detecting positions of the dancer rollers, and motors for controlling a speed of supplying the continuous web based on a position of the dancer rollers detected by the sensor are used to control the timing of supplying the continuous web.

3. The method of ejecting absorbent articles according to claim 1 or 2, wherein in the step of providing the joint portion, an accumulation device configured to accumulate and discharge the continuous web by expanding and contracting a distance between a plurality of the dancer rollers is used, and
the plurality of dancer rollers expand and contract the distance in a horizontal direction.

4. The method of ejecting absorbent articles according to any one of claims 1 to 3, wherein the absorbent articles have a central portion in a region within 30% or less of a dimension of the absorbent articles in a conveyance direction from a center of the absorbent articles.

## Patentansprüche

1. Verfahren zum Ausstoßen saugfähiger Artikel, wobei das Verfahren in einer Fertigungslinie saugfähiger Artikel verwendet wird, wobei das Verfahren enthält: einen Schritt des Anordnens von Absorptionsmitteln auf einer geförderten ununterbrochenen Bahn;
einen Schritt des Trennens der ununterbrochenen Bahn, auf der die Absorptionsmittel angeordnet sind, in jedem vorgegebenen Intervall in einer Förderrichtung des Förderns der ununterbrochenen Bahn; und
einen Schritt des Bereitstellens eines Verbindungsabschnitts, wobei ein hinteres Ende der ununterbrochenen Bahn mit einem Anfangsende einer nächsten ununterbrochenen Bahn verbunden wird, wobei das Verfahren umfasst:
einen Schritt des Steuerns einer zeitlichen Abstimmung des Zuführens der ununterbrochenen Bahn in der Weise, dass der Verbindungsabschnitt auf der Grundlage einer Länge der ununterbrochenen Bahn von dem Verbindungsabschnitt bis zu einer Trennstelle der ununterbrochenen Bahn in dem Schritt des Trennens und einer Dimension der absorbierenden Artikel in einer Förderrichtung der ununterbrochenen Bahn bei einem Mittelabschnitt der absorbierenden Artikel in einer Förderrichtung platziert wird; und
einen Schritt des Ausrückens nur absorbierender Artikel, die wenigstens den Verbindungsabschnitt von der Fertigungslinie aufweisen, und des Ausstoßens der absorbierenden Artikel während der Fertigungslinie.

2. Verfahren zum Ausstoßen absorbierender Artikel nach Anspruch 1, wobei in dem Schritt des Steuerns der zeitlichen Abstimmung des Zuführens der ununterbrochenen Bahn Tänzerwalzen, die der ununterbrochenen Bahn, die gefördert wird, Spannung verleihen, ein Sensor zum Detektieren der Positionen der Tänzerwalzen und Motoren zum Steuern einer Geschwindigkeit des Zuführens der ununterbrochenen Bahn auf der Grundlage einer durch den Sensor detektierten Position der Tänzerwalzen verwendet werden, um die zeitliche Abstimmung des Zuführens der ununterbrochenen Bahn zu steuern.

3. Verfahren zum Ausstoßen absorbierender Artikel nach Anspruch 1 oder 2, wobei in dem Schritt des Bereitstellens des Verbindungsabschnitts eine Ansammelvorrichtung verwendet wird, die dafür konfiguriert ist, die ununterbrochene Bahn durch Vergrößern und Verkleinern einer Entfernung zwischen mehreren Tänzerwalzen anzusammeln und zu entladen, und
wobei die mehreren Tänzerwalzen die Entfernung in einer horizontalen Richtung vergrößern und verkleinern.

4. Verfahren zum Ausstoßen absorbierender Artikel nach einem der Ansprüche 1 bis 3, wobei die absorbierenden Artikel einen Mittelabschnitt in einem Gebiet von höchstens 30 % einer Dimension der absorbierenden Artikel in einer Förderrichtung von einer Mitte der absorbierenden Artikel aus aufweisen.

## Revendications

1. Procédé de déchargement d'articles absorbants qui est employé dans une ligne de fabrication d'articles absorbants, comprenant : une étape d'agencement d'absorbeurs sur un rouleau continu transporté ;
une étape de découpe du rouleau continu agencé avec les absorbeurs à chaque intervalle prédéterminé dans une direction de transport du rouleau continu ; et
une étape de fourniture d'une partie jointe dans lequel une extrémité arrière du rouleau continu est jointe à une extrémité de départ d'un rouleau continu suivant, le procédé comprenant :
une étape de commande d'une synchronisation de fourniture du rouleau continu de façon à situer la partie jointe à une position centrale des articles absorbants dans une direction de transport, basée sur une longueur du rouleau continu depuis la partie jointe jusqu'à un emplacement de coupe du rouleau continu dans l'étape de coupe et une dimension des articles absorbants dans une direction de transport du rouleau continu ; et
une étape de désengagement uniquement des articles absorbants ayant au moins la partie jointe de la ligne de fabrication et de déchargement des articles absorbants au cours de la ligne de fabrication.

2. Procédé de déchargement selon la revendication 1, dans lequel dans l'étape de commande de la synchronisation de fourniture du rouleau continu, des rouleaux compensateurs qui ajoutent de la tension au rouleau continu qui est transporté, un capteur pour détecter des positions des rouleaux compensateurs, et des moteurs pour commander une vitesse de fourniture du rouleau continu basée sur une position des rouleaux compensateurs détectée par le capteur sont employés pour commander la synchronisation de fourniture du rouleau continu.

3. Procédé de déchargement selon la revendication 1 ou 2, dans lequel dans l'étape de fourniture de la partie jointe, un dispositif d'accumulation configuré pour accumuler et décharger le rouleau continu en étendant et contractant une distance entre une pluralité des rouleaux de compensation est employé, et
la pluralité de rouleaux compensateurs étend et contracte la distance dans une direction horizontale.

4. Procédé de déchargement selon l'une quelconque des revendication 1 à 3, dans lequel les articles absorbants ont une position centrale dans une zone comprise dans 30% ou moins d'une dimension des articles absorbants dans une direction de transport depuis un centre des articles absorbants.
